# EUROPEAN PATENT SPECIFICATION

(11) **EP 1 003 795 B1**
(45) Date of publication and mention of the grant of the patent: **18.02.2004**
(21) Application number: 97938179.5
(22) Date of filing: 12.08.1997
(51) Int. Cl.: C08F 220/30, G02B 1/04, C08F 220/38, C08F 220/34, C08F 220/18

(54) **OPHTHALMIC LENS POLYMERS**
POLYMERE FÜR AUGENLINSEN
POLYMERES POUR LENTILLES OPHTALMIQUES

(43) Date of publication of application: 31.05.2000
(73) Proprietor: Alcon Manufacturing Ltd., Fort Worth, Texas 76134-2099 (US)
(72) Inventor: LEBOEUF, Albert, R., Forth Worth, TX 76133 (US); KARAKELLE, Mutlu, Fort Worth, TX 76132 (US)
(74) Representative: Best, Michael, Dr.
(86) International application number: PCT/US1997/013971
(87) International publication number: WO 1999/007756

(56) References cited:
- EP-A- 0 485 197
- FR-A- 2 431 511

## Description

### FIELD OF THE INVENTION

This invention relates to polymers and their use in ophthalmic lenses, particularly intraocular lenses that can be inserted through small incisions.

### BACKGROUND OF THE INVENTION

In response to the development of cataractous lenses, it has become common to replace the lens with an intraocular lens (IOL) in a surgical procedure. In order to reduce the trauma to the eye in cataract surgery, it is desirable to keep the incision through which the surgical procedure is conducted as small as possible. With the development of phacoemulsification surgery, in which the lens is fragmented by ultrasonic vibrations and the fragments aspirated through a small cannula, it has become possible to remove a lens through an incision no larger than 2-3 millimeters. However, since an IOL is typically at least six millimeters in diameter, an incision at least that large has to be made to permit the insertion of the IOL. In order to permit the use of the desirable small incision technique, various flexible, distortable, and inflatable IOLs have been devised.

Juergens, U.S. Patent No. 4,619,662, discloses a collapsible intraocular lens with a hollow interior which can be evacuated to cause the lens to collapse to a relatively small size. The collapsed lens can then be inserted into the eye through a relatively small incision. After insertion, the interior of the lens is filled with an elastomer to expand the lens to the proper shape and dimension.

Mazzocco, U.S. Patent No. 4,573,998, discloses a deformable intraocular lens that can be rolled, or folded to fit through a relatively small incision. The deformable lens is inserted while it is held in its distorted configuration, then released inside the chamber of the eye, whereupon the elastic property of the lens causes it to resume its molded shape. As suitable materials for the deformable lens, Mazzocco discloses polyurethane elastomers, silicone elastomers, hydrogel polymer compounds, organic or synthetic gel compounds and combinations thereof.

Keates et al., U.S. Patent No. 4,619,657, disclose a flexible intraocular lens holder made from a flexible inert polymer, such as silicone rubber, which contains pockets for receiving individual lenses which are small enough to fit through a relatively small incision. The lens holder is folded or rolled and inserted through a small incision and thereafter several of the small lenses are inserted through the incision and into the pockets in the lens holder to form a composite intraocular lens.

Gasser et al., U.S. Patent No. 5,224,957, disclose photopolymerizable compositions useful in forming an intraocular lens in situ. The compositions are delivered into the natural lens capsule or a this plastic shell substitute and then polymerized. The reference compositions contain 90 - 99.99% by weight of at least one at least difunctional acrylic and/or methacrylic acid ester. Suitable acid esters include bisphenol A or bishydroxypolyalkoxy bisphenol A derivatives lengthened with ethylene oxide or propylene oxide. Preferred acrylic and/or methacrylic acid esters include those having the formula:

CH₂=CH-COO-(CH₂(CH₂)ₓO)ₙ-phenyl-C(CH₃)₂-phenyl-(O(CH₂)_{y}CH₂)ₘ-OOC-CH=CH₂,

wherein n, m = 1-5, and x, y = 1-3.
FR-A-2 431 511 discloses an organic glass copolymer from which lenses having an excellent surface hardness and impact resistance can be prepared.

There is a need for a material, with a relatively high refractive index, that can be used to form a flexible intraocular lens capable of being simply rolled or folded for insertion through a small incision.

### SUMMARY OF THE INVENTION

This invention is directed to high refractive index copolymers consisting essentially of (i) a total of at least 60% (wt.) of one or more monomers having the structure: 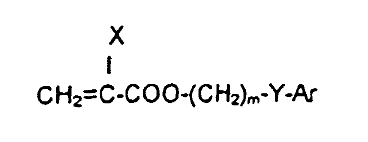 wherein:
X is H;
m is 0-10;
Y is nothing, O, S, or NR wherein R is H, CH₃, CₙH₂ₙ₊₁ (n=1-10) iso OC₃H₇, C₆H₅, or CH₂C₆H₅;
Ar is any aromatic ring which can be unsubstituted or substituted with H, CH₃, C₂H₅, n-C₃H₇, iso-C₃H₇, OCH₃, C₆H₁₁, Cl, Br, C₆H₅, or CH₂C₆H₅;
and (ii) a total of at least 10% (wt.) of one or more monomers: having the structure: 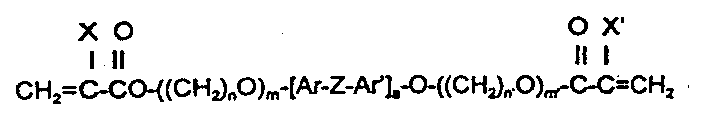 wherein:
X, X' are H;
n, n' are independently 2 or 3;
m, m' are independently 2 - 25;
Ar, Ar' are independently as defined above;
a is 1 or 2; and
Z is C(CH₃)₂ or S(=O)₂; provided that the copolymer has a Tg no greater than 20°C and an elongation of at least 150%; and optionally of further ingredients selected from the group consisting of polymerization initiators, copolymerizable cross-linking monomers, and copolymerizable UV- and blue-light-blocking chromophores.

These copolymers can be used to form various types of ophthalmic lenses, including. but not limited to, intraocular lenses, contact lenses, spectacle lenses, lenses for optical instruments. For example, the copolymers defined above may be used to form intraocular lenses that have high refractive indexes, are flexible and transparent, can be inserted into the eye through a relatively small incision, and recover their original shape after insertion.

### BRIEF DESCRIPTION OF THE DRAWINGS

Figure 1 shows a multipiece intraocular lens.
Figure 2 shows a cross-section of a mold for making intraocular lenses.

### DETAILED DESCRIPTION OF THE INVENTION AND PREFERRED EMBODIMENTS

The high refractive index copolymers of the present invention consist essentially of (i) a total of at least 60% (wt.) of one or more monomers having the structure: 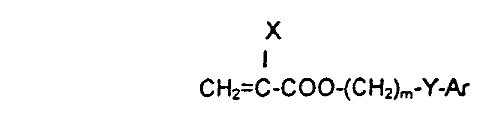 wherein:
X is H;
m is 0-10;
Y is nothing. O, S, or NR wherein R is H, CH₃, CₙH₂ₙ₊₁ (n=1-10) iso OC₃H₇, C₆H₅, or CH₂C₆H₅;
Ar is any aromatic ring which can be unsubstituted or substituted with H, CH₃, C₂H₅, n-C₃H₇, iso-C₃H₇, OCH₃, C₆H₁₁, Cl, Br, C₅H₅, or CH₂C₆H₅;
and (ii) a total of at least 10% (wt.) of one or more monomers having the structure: 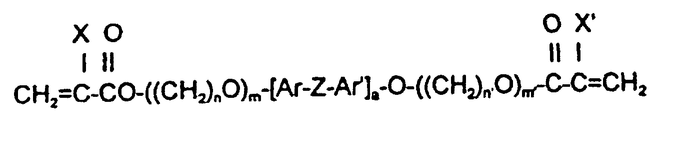 wherein:
X, X' are H;
n, n' are independently 2 or 3:
m, m' are independently 2 - 25;
Ar, Ar' are independently as defined above;
a is 1 or 2; and
Z is C(CH₃)₂ or S(=O)₂.

Type (i) monomers are known and include 2-phenoxyethyl acrylate, 2-phenylethylthio acrylate, 2-phenylethylamino acrylate, phenyl acrylate, benzyl acrylate, 2-phenylethyl acrylate, 3-phenylpropyl acrylate, 4-phenylbutyl acrylate, 4-methylphenyl acrylate, 4-methylbenzyl acrylate, 2-2-methylphenylethyl acrylate, 2-3-methylphenylethyl acrylate, 2-4-methylphenylethyl acrylate, and the like. These acrylic monomers and others are disclosed in U.S. Patent No. 5,290,892.

Preferred monomers of type (i) are those where X is H; m is 2-4; Y is nothing or O; and Ar is phenyl. Most preferred are 2-phenylethyl acrylate, 2-phenoxyethyl acrylate, 3-phenylpropyl acrylate, 3-phenoxypropyl acrylate, 4-phenylbutyl acrylate, and 4-phenoxybutyl acrylate.

The amount of type (i) monomer present in the high refractive index copolymers of the present invention will vary depending upon the identity of the type (i) monomer(s), the identity of the type (ii) monomer(s) and the mechanical properties desired for the final copolymer. Foldable intraocular lenses are made from copolymers having a glass transition temperature no greater than 20°C, in order that the lenses can be rolled or folded conveniently at room temperature. Additionally, copolymers exhibiting an elongation of at least 150% are used in foldable intraocular lenses because such lenses must exhibit sufficient strength to allow them to be folded without fracturing. Preferred for foldable intraocular lens applications are polymers having an elongation of at least 200%. Thus, for foldable intraocular lens applications, the copolymers of the present invention will contain at least 60% by weight, preferably at least about 80% by weight, of the type (i) monomer(s).

Type (ii) monomers can be synthesized using known methods and many are commercially available from a variety of sources (e.g., Dajac Laboratories, Inc. (Feastervilte, PA). Preferred type (ii) monomers are those where n and n' are 2; m and m' are independently 2 - 12; Ar and Ar' are phenyl; Z is C(CH₃)₂; and a is 1. Most preferred is the type (ii) monomer where X=X'=H, n=n'=2, m=m'=2, Ar=Ar'=phenyl, a=1, and Z=C(CH₃)₂ [hereinafter referred to as "Ethoxylated (4 moles) bisphenol A diacrylate"].

As in the case of the monomer of type (i), the amount of type (ii) monomer present in the in the high refractive index copolymers of the present invention will vary depending upon the identity of the type (i) monomer(s), the identity of the type (ii) monomer(s), and the mechanical properties desired for the final copolymer. In general, for foldable intraocular lens applications, the copolymers of the present invention will contain from 10 to 40% by weight, preferably from 10 to 20% by weight, of the type (ii) monomer(s).

For foldable intraocular lens applications m and m' in the type (ii) monomer will be relatively small in order to achieve a flexible, foldable copolymeric material.

Although not essential, the copolymers of the present invention may optionally contain one or more of a variety of other ingredients selected from polymerization initiators, copolymerizable cross-linking monomers, and copolymerizable UV- or blue-light blocking chromophores. Polymerization initiators may be, for example, thermal or light-activated initiators.

Typical thermal free radical initiators include peroxides, such as benzophenone peroxide, peroxycarbonates, such as bis-(4-t-butylcyclohexyl) peroxydicarbonate, azonitriles, such as azobisisobytyronitrile, and the like. Preferred initiators are bis-(4-t-butylcyclohexyl) peroxydicarbonate and t-butyl peroxy-2-ethyl hexanoate ("t-butyl-peroctoate"). Alternatively, the monomers can be photopolymerized by using a mold which is transparent to radiation of a wavelength capable of initiating polymerization of these acrylic monomers by itsetf. For materials lacking UV-absorbing chromophores, conventional photoinitiator compounds, e.g., a benzophenone-type photoinitiator, can also be introduced to facilitate the polymerization. Photosensitizers can be introduced as well to permit the use of longer wavelengths; however, in preparing a polymer which is intended for long residence within the eye, it is generally preferable to keep the number of ingredients in the polymer to a minimum to avoid the presence of materials which might leach from the lens into the interior of the eye.

If desired, suitable cross-linking monomers include almost any terminally ethylenically unsaturated compound having more than one unsaturated group. Suitable cross-linking agents include, for example: ethylene glycol dimethacrylate, diethylene glycol dimethacrylate, allyl methacrylate, 1,3-propanediol dimethacrylate, allyl methacrylate, 1,6-hexanediol dimethacrylate, 1,4-butanediol. dimethacrylate, and the like. A preferred cross-linking agent is 1,4-butanediol diacrylate (BDDA).

Ultraviolet and/or blue-light absorbing chromophores can also be included in the copolymers of the present invention, as may be desirable in the case where the copolymers are used to make intraocular lenses. Such chromophores allow the light absorbance of an intraocular lens to approximate that of the eye's natural lens. The ultraviolet absorbing material can be any compound which absorbs ultraviolet light, i.e., light having a wavelength shorter than about 400 nm, but does not absorb any substantial amount of visible light. The ultraviolet absorbing compound is incorporated into the monomer mixture and is entrapped in the polymer matrix when the monomer mixture is polymerized. Suitable ultraviolet absorbing compounds include substituted benzophenones, such as 2-hydroxybenzophenone, and 2-(2-hydroxyphenyl)-benzotriazoles. It is preferred to use an ultraviolet absorbing compound which is copolymerizable with the type (i) and (ii) monomers described above and is thereby covalently bound to the polymer matrix In this way possible leaching of the ultraviolet absorbing compound out of the lens and into the interior of the eye is minimized. Suitable copolymerizable ultraviolet absorbing compounds are the substituted 2-hydroxybenzophenones disclosed in U.S. Patent No. 4,304,895 and the 2-hydroxy-5-acrylocyphenyl-2H-benzotriazoles disclosed in U.S. Patent No. 4,528,311. The most preferred ultraviolet absorbing compound is 2-(3'-methallyl-2'-hydroxy-5'-methyl phenyl) benzotriazole.

As in the case of UV-absorbers, it is preferred to use blue-light absorbing compounds which are coplymerizable with the type (i) and type (ii) monomers. Suitable polymerizable blue-light blocking chromophores include those disclosed in U.S. Patent No. 5,470,932.

The copolymers of this invention are prepared by generally conventional polymerization methods. For example, a mixture of the liquid monomers in the desired proportions together with a conventional thermal free-radical initiator is prepared. The mixture can then be introduced into a mold of suitable shape to form an ophthalmic lens, and the polymerization carried out by gentle heating to activate the initiator.

Intraocular lenses (IOLs) constructed of the disclosed polymers can be of any design capable of being rolled or folded into a small cross section that can fit through a relatively smaller incision. For example, the IOLs can be of what is known as a one piece or multipiece design. Typically, an IOL comprises an optic and at least one haptic. The optic is that portion which serves as the lens and the haptics are attached to the optic and are like arms which hold the optic in its proper place in the eye. The optic and haptic(s) can be of the same or different material. A multipiece lens, as shown in Figure 1, is so called because the optic and the haptic(s) are made separately and then the haptics are attached to the optic. Haptics may be attached to the optic using conventional techniques. in a single piece lens, the optic and the haptics are formed out of one piece of material. Depending on the material, the haptics are then cut, or lathed, out of the material to produce the IOL.

Molding and drilling operations are easily carried out if the optic is molded between two polypropylene mold halves as shown in Figure 2. The mold containing the cured optic material is then placed on a lathe and the desired optic diameter is lathe cut. The mold may then be easily mounted to carry out any drilling operations prior to removing the mold halves. Both the lathing and drilling operations may be facilitated by cooling the mold/optic in a freezer to less than 10°C and preferably less than 0°C prior to each of these operations.

The invention will be further illustrated by the following examples which are intended to be illustrative.

### EXAMPLES

The mixtures of Examples 1 - 3, shown in Table 1 below, were each transferred into two molds: (i) an IOL mold of the type illustrated in Figure 1 and (ii) a slab mold made of two polypropylene plates. The filled molds were clamped with spring clamps and cured in an oven for 2 hours at 80 °C and 2 hours at 110°C. At the end of the polymerization period, the molds were allowed to cool to room temperature. The molds were then opened and the cured intraocular lens and sheet of polymer were removed.

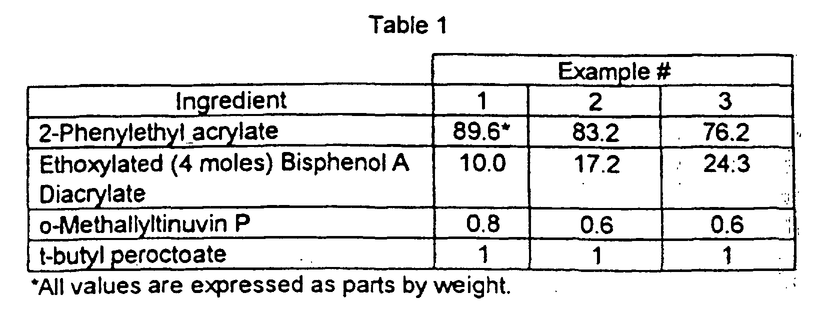

The physical properties of the cured lenses and slabs, shown in Table 2 below, were analyzed (room temperature conditions) as follows: the glass transition temperature (T_{g}) was measured by differential thermal analysis using conventional equipment. The Secant Modulus (psi), tensile strength (psi), and ultimate elongation (% strain) were determined using an Instron Model 1122 Material Tester for a dumbell-shaped sample of material. The refractive index was measured with an Abbe refractometer.

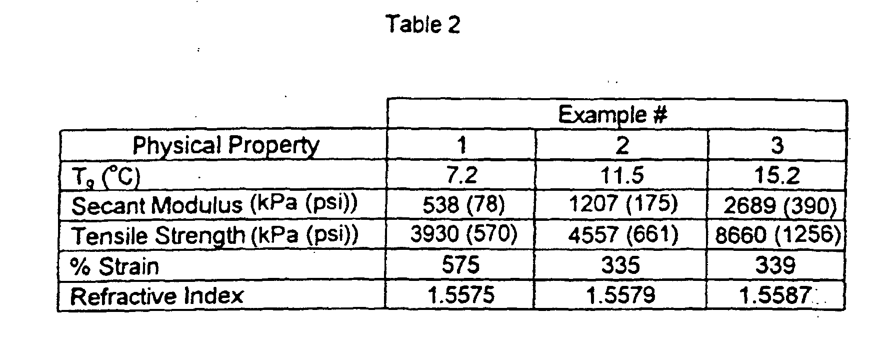

## Claims

1. A copolymer consisting essentially of (i) a total of at; least 60% (wt.) of one or more monomers having the structure: 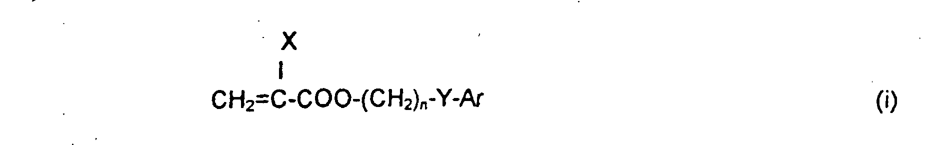 wherein:
X is H ;
n is 0-10;
Y is nothing, O, S, or NR wherein R is H, CH₃, CₙH₂ₙ₊₁ (n=1-10) iso OC₃H₇, C₆H₅, or CH₂C₆H₅;
Ar is any aromatic ring which can be unsubstituted or substituted with H, CH₃, C₂H₅, n-C₃H₇, iso-C₃H₇, OCH₃, C₆H₁₁, Cl, Br, C₆H₅, or CH₂C₆H₅;
and(ii) a total of at least 10% (wt.) of one or more monomers having the structure: 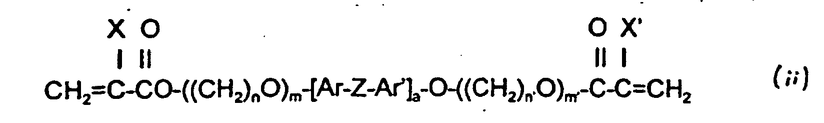 wherein:
X, X' are H;
n, n' are independently 2 or 3;
m, m' are independently 2 - 25;
Ar, Ar' are independently as defined above;
a is 1 or 2; and
Z is C(CH₃)₂ or S(=O)₂ ; provided that the copolymer has: a Tg no greater than 20°C and an elongation of at least 150%;
and optionally of further ingredients selected from the group consisting of polymerization initiators, copolymerizable cross-linking monomers, and copolymerizable UV- and blue-light-blocking chromophores.

2. The copolymer of Claim 1 wherein the monomer(s) of structure (i) have n = 2-4; Y = nothing or O; and Ar = phenyl.

3. The copolymer of Claim 2 wherein the monomer(s) of structure (i) are selected from the group consisting of 2-phenylethyl acrylate, 2-phenoxyethyl acrylate, 3-phenylpropyl acrylate, 3-phenoxypropyl acrylate, 4-phenylbutyl acrylate, and 4-phenoxybutyl acrylate.

4. The copolymer of Claim 1 wherein the monomer(s) of structure (ii) have n and n' = 2; m and m' independently = 2 - 12; Ar and Ar' = phenyl; Z = C(CH₃)₂; and a is 1.

5. The copolymer of Claim 4 wherein m and m' = 2.

6. The copolymer of Claim 2 wherein the monomer(s) of structure (ii) have n and n' = 2; m and m' independently = 2 - 12; Ar and Ar' = phenyl; Z = C(CH₃)₂; and a is 1.

7. The copolymer of Claim 1 wherein the total amount of the monomer(s) of structure (i) is at least about 80% (wt.).

8. An ophthalmic lens consisting, essentially of (i) a total of at least 60% (wt.) of one or more monomers having the structure: 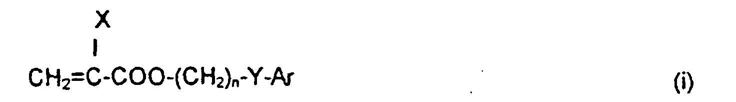 wherein:
X is H ;
n is 0-10;
Y is nothing, O, S, or NR wherein R is H, CH₃, CₙH₂ₙ₊₁ (n=1-10) iso OC₃H₇, C₆H₅, or CH₂C₆H₅;
Ar is any aromatic ring which can be unsubstituted or substituted with H, CH₃, C₂H₅, n-C₃H₇, iso-C₃H₇, OCH₃, C₆H₁₁, Cl, Br, C₆H₅, or CH₂C₆H₅;
and (ii) a total of at least 10% (wt.) of one or more monomers having the structure: 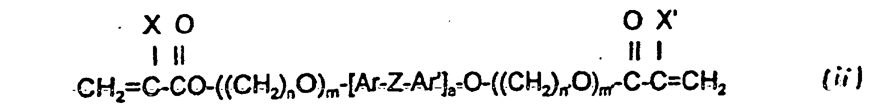 wherein:
X, X' are H;
n, n' are independently 2 or 3;
m, m' are independently 2 - 25;
Ar, Ar' are independently as defined above;
a is 1 or 2; and,
Z is C(CH₃)₂ or S(=O)₂; provided that the copolymer has a Tg no greater than 20°C and an elongation of at least 150 %
and optionally of further ingredients selected from the group consisting of polymerization initiators, copolymerizable cross-linking monomers, and copolymerizable UV- and blue-light-blocking chromophores.

9. The ophthalmic lens of Claim 8 wherein the monomer(s) of structure (i) have n = 2-4; Y = nothing or O; and Ar = phenyl.

10. The ophthalmic lens of Claim 9 wherein the monomer(s) of structure (i) are selected from the group consisting of 2-phenylethyl acrylate, 2-phenoxyethyl acrylate, 3-phenylpropyl acrylate, 3-phenoxypropyl acrylate, 4-phenylbutyl acrylate, and 4-phenoxybutyl acrylate.

11. The ophthalmic lens of Claim 8 wherein the monomer(s) of structure (ii) have n and n' = 2; m and m' independently = 2 - 12; Ar and Ar' = phenyl; Z = C(CH₃)₂; and a is 1.

12. The ophthalmic lens of Claim 11 wherein m and m'= 2.

13. The ophthalmic lens of Claim 9 wherein the monomer(s) of structure (ii) have n and n' = 2; m and m' independently = 2 - 12; Ar and Ar' = phenyl; Z = C(CH₃)₂; and a is 1.

14. The ophthalmic lens of Claim 8 wherein the total amount of the monomer(s) of structure (i) is at least about 80% (wt.).

15. The ophthalmic lens of Claim 8 wherein the ophthalmic lens is an intraocular lens optic.

## Patentansprüche

1. Copolymer im Wesentlichen bestehend aus (i) insgesamt mindestens 60% (Gew.) eines oder mehrerer Monomere mit der Struktur: 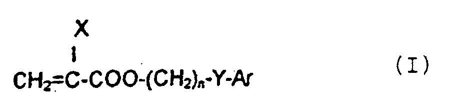 worin
X H ist;
n 0 bis 10 ist;
Y nichts, O, S oder NR ist, worin R H, CH₃, CₙH₂ₙ₊₁ (n = 1 bis 10) iso OC₃H₇, C₆H₅ oder CH₂C₆H₅ ist;
Ar irgendein aromatischer Ring ist, der unsubstituiert oder mit H, CH₃, C₂H₅, n-C₃H₇, iso-C₃H₇, OCH₃, C₆H₁₁, Cl, Br, C₆H₅ oder CH₂C₆H₅ substituiert sein kann;
und (ii) insgesamt mindestens 10% (Gew.) eines oder mehrerer Monomere mit der Struktur: 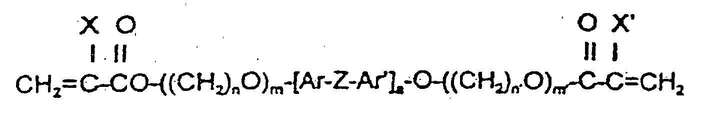 worin
X, X' H sind;
n, n' unabhängig 2 oder 3 sind;
m, m' unabhängig 2 bis 25 sind;
Ar, Ar' unabhängig wie oben definiert sind;
a 1 oder 2 ist und
Z C(CH₃)₂ oder S(=O)₂ ist, mit dem Vorbehalt, dass das Copolymer eine Tg von nicht mehr als 20°C und eine Dehnung von mindestens 150% hat und
gegebenenfalls weiteren Inhaltsstoffen, die ausgewählt sind aus der Gruppe bestehend aus Polymerisationsinitiatoren, copolymerisierbaren vemetzenden Monomeren und copolymerisierbaren UV- und Blaulicht blockierenden Chromophoren.

2. Copolymer nach Anspruch 1, wobei bei dem/den Monomer(en) der Struktur (i) n = 2 bis 4; Y = nichts oder O und Ar = Phenyl.

3. Copolymer nach Anspruch 2, wobei das Monomer/die Monomeren der Struktur (i) ausgewählt sind aus der Gruppe bestehend aus 2-Phenylethylacrylat, 2-Phenoxyethylacrylat, 3-Phenylpropylacrylat, 3-Phenoxypropylacrylat, 4-Phenylbutylacrylat und 4-Phenoxybutylacrylat.

4. Copolymer nach Anspruch 1, wobei bei dem/den Monomer(en) der Struktur (ii) n und n' = 2; m und m' unabhängig = 2 bis 12; Ar und Ar' = Phenyl; Z = C(CH₃)₂ und a = 1.

5. Copolymer nach Anspruch 4, wobei m und m' = 2.

6. Copolymer nach Anspruch 2, wobei bei dem/den Monomer(en) der Struktur (ii) n und n' = 2; m und m' unabhängig = 2 bis 12; Ar und Ar' = Phenyl; Z = C(CH₃)₂ und a = 1.

7. Copolymer nach Anspruch 1, wobei die Gesamtmenge des/der Monomeren der Struktur (i) mindestens etwa 80% (Gew.) ist.

8. Ophthalmische Linse bestehend im Wesentlichen aus (i) mindestens 60% (Gew.) eines oder mehrerer Monomere mit der Struktur: 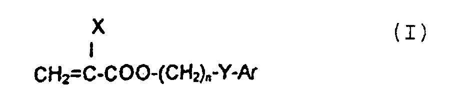 worin
X H ist;
n 0 bis 10 ist;
Y nichts, O, S oder NR ist, worin R H, CH₃, CₙH₂ₙ₊₁ (n = 1 bis 10) iso OC₃H₇, C₆H₅ oder CH₂C₆H₅ ist;
Ar irgendein aromatischer Ring ist, der unsubstituiert oder mit H, CH₃, C₂H₅, n-C₃H₇, iso-C₃H₇, OCH₃, C₆H₁₁, Cl, Br, C₆H; oder CH₂C₆H₅ substituiert sein kann;
und (ii) insgesamt mindestens 10% (Gew.) eines oder mehrerer Monomere mit der Struktur: 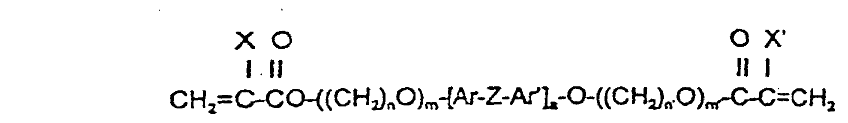 worin
X, X' H sind;
n, n' unabhängig 2 oder 3 sind;
m, m' unabhängig 2 bis 25 sind;
Ar, Ar' unabhängig wie oben definiert sind;
a 1 oder 2 ist und
Z C(CH₃)₂ oder S(=O)₂ ist, mit dem Vorbehalt, dass das Copolymer eine Tg von nicht mehr als 20°C und eine Dehnung von mindestens 150% hat und
gegebenenfalls weiteren Inhaltsstoffen, die ausgewählt sind aus der Gruppe bestehend aus Polymerisationsiniriatoren, copolymerisierbaren vemetzenden Monomeren und copolymerisierbaren UV- und Blaulicht blockierenden Chromophoren.

9. Ophthalmische Linse nach Anspruch 8, wobei bei dem/den Monomer(en) der Struktur (i) n = 2 bis 4; Y = nichts oder O und Ar = Phenyl.

10. Opthalmische Linse nach Anspruch 9, wobei das Monomer/die Monomeren der Struktur (i) ausgewählt sind aus der Gruppe bestehend aus 2-Phenylethylacrylat, 2-Phenoxyethylacrylat, 3-Phenylpropylacrylat, 3-Phenoxypropylacrylat, 4-Phenylbutylacrylat und 4-Phenoxybutylacrylat.

11. Ophthalmische Linse nach Anspruch 8, wobei bei dem/den Monomer(en) der Struktur (ii) n und n' = 2; m und m' unabhängig = 2 bis 12; Ar und Ar' = Phenyl; Z = C(CH₃)₂ und a = 1.

12. , Ophthalmische Linse nach Anspruch 11, worin m und m' = 2.

13. Ophthalmische Linse nach Anspruch 9, wobei bei dem/den Monomer(en) der Struktur (ii) n und n' = 2; m und m' unabhängig = 2 bis 12; Ar und Ar' = Phenyl; Z = C(CH₃)₂ und a = 1.

14. Ophthalmische Linse nach Anspruch 8, wobei die Gesamtmenge des/der Monomer(en) der Struktur (i) mindestens etwa 80% (Gew.) ist.

15. Ophthalmische Linse nach Anspruch 8, wobei die ophthalmische Linse eine Intraokularlinsenoptik ist.

## Revendications

1. Copolymère formé essentiellement (i) d'un total d'au moins 60% (poids) d'un ou plusieurs monomères ayant la structure : 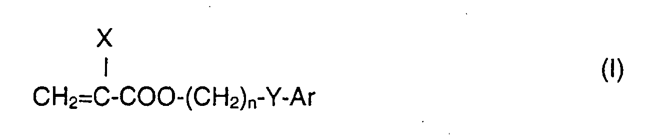 dans laquelle
X est H;
n est égal à 0-10;
Y représente rien, O, S ou NR, R représentant H, CH₃, CₙH₂₊₁ (n=1-10), iso-OC₃H₇, C₆H₅ ou CH₂C₆H₅;
Ar est un noyau aromatique quelconque qui peut être non substitué ou substitué par H, CH₃, C₂H₅, n-C₃H₇, iso-C₃H₇, OCH₃, C₆H₁₁, Cl, Br, C₆H₅ ou CH₂C₆H₅;
et (ii) d'un total d'au moins 10% (poids) d'un ou plusieurs monomères ayant la structure : 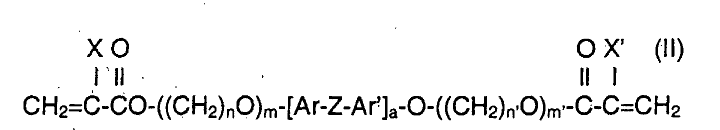 dans laquelle :
X, X' sont H;
n, n' sont indépendamment égaux à 2 ou 3;
m, m' sont indépendamment égaux à 2-25.
Ar, Ar' sont indépendamment tels que définis précédemment;
a est égal à 1 ou 2; et
Z est C(CH₃) ou S(=O)₂; pour autant que le copolymère ait une Tg non supérieure à 20°C et un allongement d'au moins 150%; et éventuellement d'autres ingrédients choisis dans le groupe comprenant les initiateurs de polymérisation, les monomères de réticulation copolymérisables et les chromophores bloquant la lumière U.V. et bleue copolymérisables.

2. Copolymère suivant la revendication 1, dans lequel le(s) monomère(s) de structure (i) ont n=2-4; Y=rien ou O; et Ar=phényle.

3. Copolymère suivant la revendication 2, dans lequel le(s) monomère(s) de structure (i) sont choisis dans le groupe comprenant l'acrylate de 2-phényléthyle, l'acrylate de 2-phénoxyéthyle, l'acrylate de 3-phénylpropyle, l'acrylate de 3-phénoxypropyle, l'acrylate de 4-phényl-butyle et l'acrylate de 4-phénoxybutyle.

4. Copolymère suivant la revendication 1, dans lequel le(s) monomère(s) de structure (ii) ont n et n'=2; m et m' indépendamment =2-12; Ar et Ar'=phényle; Z=C(CH₃)₂; et a est égal à 1.

5. Copolymère suivant la revendication 4, dans lequel m et m'=2.

6. Copolymère suivant la revendication 2, dans lequel le(s) monomère(s) de structure (ii) ont n et n'=2; m et m' indépendamment =2-12; Ar et Ar' = phényle; Z=C(CH₃)₂; et a est égal à 1.

7. Copolymère suivant la revendication 1, dans lequel la quantité totale du ou des monomères de structure (i) est d'au moins environ 80% (poids).

8. Lentille ophtalmique formée essentiellement (i) d'un total d'au moins 60% (poids) d'un ou plusieurs monomères ayant la structure : 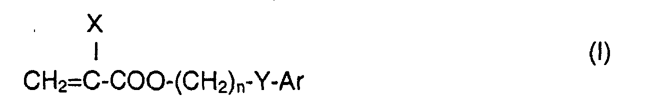 dans laquelle :
X est H;
n est égal à 0-10;
Y représente rien, O, S ou NR, R représentant H, CH₃, CₙH₂₊₁ (n=1-10), iso-OC₃H₇, C₆H₅ ou CH₂C₆H₅;
Ar est un noyau aromatique quelconque qui peut être non substitué ou substitué par H, CH₃, C₂H₅, n-C₃H₇, iso-C₃H₇, OCH₃, C₆H₁₁, Cl, Br, C₆H₅ ou CH₂C₆H₅;
et (ii) d'un total d'au moins 10% (poids) d'un ou plusieurs monomères ayant la structure : 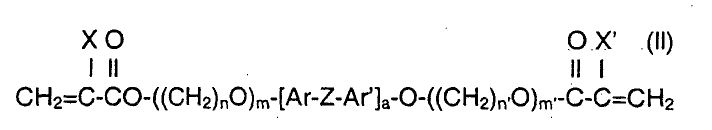 dans laquelle :
X, X' sont H;
n, n' sont indépendamment égaux à 2 ou 3;
m, m' sont indépendamment égaux à 2-25.
Ar, Ar' sont indépendamment tels que définis, précédemment;
a est égal à 1 ou 2; et
Z est C(CH₃) ou S(=O)₂; pour autant que le copolymère ait une Tg non supérieure à 20°C et un allongement d'au moins 150%; et éventuellement d'autres ingrédients choisis dans le groupe comprenant les initiateurs de polymérisation, les monomères de réticulation copolymérisables et les chromophores bloquant la lumière U.V. et bleue copolymérisables.

9. Lentille ophtalmique suivant la revendication 8, dans laquelle le(s) monomère(s) de structure (i) ont n=2-4; Y=rien ou O; et Ar=phényle.

10. Lentille ophtalmique suivant la revendication 9, dans laquelle le(s) monomère(s) de structure (i) sont choisis dans le groupe comprenant l'acrylate de 2-phényléthyle, l'acrylate de 2-phénoxyéthyle, l'acrylate de 3-phénylpropyle, l'acrylate de 3-phénoxypropyle, l'acrylate de 4-phénylbutyle et l'acrylate de 4-phénoxybutyle.

11. Lentille ophtalmique suivant la revendication 8, dans laquelle le(s) monomère(s) de structure (ii) ont n et n'=2; m et m' indépendamment = 2-12; Ar et Ar' = phényle; Z=C(CH₃)₂; et a est égal à 1.

12. Lentille ophtalmique suivant la revendication 11, dans laquelle m et m'=2.

13. Lentille ophtalmique suivant la revendication 9, dans laquelle le(s) monomère(s) de structure (ii) ont n et n'=2; m et m' indépendamment =2-12; Ar et Ar'=phényle; Z=C(CH₃)₂; et a est égal à 1.

14. Lentille ophtalmique suivant la revendication 8, dans laquelle la quantité totale du ou des monomères de structure (i) est d'au moins environ 80% (poids).

15. Lentille ophtalmique suivant la revendication 8, dans laquelle la lentille ophtalmique est une optique de lentille intraoculaire.
